# EUROPÄISCHE PATENTSCHRIFT

(11) **EP 1 573 327 B1**
(45) Veröffentlichungstag und Bekanntmachung des Hinweises auf die Patenterteilung: **16.10.2013**
(21) Anmeldenummer: 03795750.3
(22) Anmeldetag: 15.12.2003
(51) Int. Cl.: G01N 33/543, C12Q 1/68

(54) **Dna-chip mit einem Mikroarray aus Mikroelektrodensystemen**
Dna chip comprising a microarray made of microelectrode systems
Puce a adn comportant un microreseau constitué de systèmes de microélectrodes

(30) Priorität: 19.12.2002 DE 10259820
(43) Veröffentlichungstag der Anmeldung: 14.09.2005
(73) Patentinhaber: Siemens Aktiengesellschaft, 80333 München (DE)
(72) Erfinder: SCHIENLE, Meinrad, 85521 Ottobrunn (DE); THEWES, Roland, 82194 Gröbenzell (DE); GUMBRECHT, Walter, 91074 Herzogenaurach (DE); MUND, Konrad, 91080 Uttenreuth (DE)
(86) Internationale Anmeldenummer: PCT/DE2003/004127
(87) Internationale Veröffentlichungsnummer: WO 2004/057334

(56) Entgegenhaltungen:
- WO-A-00/62047
- WO-A-00/63682
- WO-A-02/42759
- WO-A-90/12314
- DE-A- 19 610 115

## Beschreibung

Die Erfindung betrifft einen DNA-Chip mit einem Mikroarray aus Mikroelektrodensystemen entsprechend dem Patentanspruch 1.

Ein DNA-Chip umfasst allgemein einen meist flach ausgebildeten Träger, auf dem ein Mikroarray aus Spots angeordnet ist. Ein Spot enthält auf den Oberflächen von Träger und Elektroden immobilisierte Fängermoleküle, beispielsweise Oligonucleotide. Zur Durchführung einer Analyse wird auf die Spots eine Zielmoleküle, beispielsweise DNA-Bruchstücke, enthaltende Analytlösung aufgebracht. Im Falle einer komplementären Übereinstimmung in der Basensequenz koppeln die Zielmoleküle an die Fängermoleküle eines Spots an. Die Auslesung des Analysenergebnisses, also die Bestimmung jener Spots, in denen Kopplungs- bzw. Bindungsereignisse stattgefunden haben, kann beispielsweise optisch, kalorimetrisch oder elektrisch erfolgen.

Aus der DE 196 10 115 C2 und der Veröffentlichung "Nanoscaled interdigitated electrode arrays for biochemical sensors", Van Gerwen et al., Sensors and Actuators, B49, 1998, 73-80, Elsevier Science S.A. sind elektrisch auslesbare DNA-Chips bekannt, die zur elektrischen Detektion von Bindungsereignissen zweipolige Mikroelektrodensysteme aufweisen. Diese sind jeweils aus einem Paar kammartiger, miteinander verzahnter Elektroden gebildet, welche mit Wechselstrom beaufschlagt sind. Im Bereich der Elektroden stattfindende Bindungsereignisse verändern elektrische Parameter wie z.B. den Leitwert und die spezifische Kapazität des Analyten und sind dementsprechend mit Hilfe des Mikroelektrodensystems detektierbar.

Problematisch bei derartigen DNA-Chips ist, dass die Dimension der Elektroden sehr groß ist im Vergleich zu den molekularen Dimensionen der in monomolekularer Lage auf der Träger- und Elektrodenoberfläche vorhandenen Fängermoleküle. Dort stattfindende Bindungsereignisse sind daher nur schwer detektierbar. Bei van Gerwen et al wird zur Verbesserung des Messeffektes bzw. der Sensitivität eine Verkleinerung der Elektroden vorgeschlagen. Einer Miniaturisierung sind jedoch aus technischen und ökonomischen Gründen Grenzen gesetzt.

Ein weiteres Problem besteht darin, dass beim Nachweis von biochemischen Molekülen relativ hohe Elektrolytleitwerte und dementsprechend niedrige Analytwiderstände vorliegen. Diese werden von der meist sehr hohen, durch die Elektrolyt-Doppelschichtkapazität zwischen Elektroden und Analyt hervorgerufene, Elektrodenimpedanz überlagert. Eine Separierung von Analytwiderstand und Elektrodenimpedanz ist nahezu unmöglich. Außerdem sind aufgrund des kleinen Analytwiderstandes sehr hohe Messfrequenzen nötig. Dies ist mit herkömmlicher Messtechnik jedoch sehr schwierig, da parasitäre Kapazitäten, wie Kabelkapazitäten etc., die Messung stören.

Bei herkömmlichen DNA-Chips sind also die Messeffekte zur Ermittlung der Kapazität bzw. des Widerstands des Analyten sehr schwach ausgeprägt oder nicht vorhanden. Darüber hinaus müssen die Messfrequenzen im MHz-Bereich liegen. Außerdem beeinflussen alle chemischen oder physikalischen Vorgänge, die an den Elektroden stattfinden, die Messung zwischen den Elektroden, so z.B. Belegungen mit biochemischen Molekülen, Polarisierungen, Korrosion der Elektroden, Filmbildung usw.

Weiterhin ist aus DE 100 15 816 A1 ein elektrisch auslesbarer DNA-Chip bekannt, bei dem ein Zweipol-Elektrodensystem zum elektrischen Monitoring eines Redox-Cycling-Prozesses ermöglicht wird. Gegebenenfalls kann dazu weiterhin eine dritte Elektrode mit Potentialbeaufschlagung zur Steuerung des Redox-Cycling-Prozesses vorhanden sein.

Das Dokument WO00/62047 beschreibt einen DNA-Chip mit einem Flachträger und einem darauf angeordneten Mikroarray von immobilisierte Fängermoleküle enthaltenden Spots, wobei jeder Spot ein MikroelektrodenSystem zur impedanzspektroskopischen Detektion von Bindungsereignissen zwischen den Fängermolekülen und Zielmolekülen einer auf die Spots applizierten Analytlösung enthält und wobei das Mikroelektrodensystem ein Dünnschicht-Vierpolsystem ist.

Ausgehend vom Stand der Technik ist es Aufgabe der Erfindung, einen verbesserten elektrisch auslesbaren DNA-Chip zu schaffen, mit dem die Erfassung von analytspezifischen Messwerten erleichtert ist.

Diese Aufgabe wird durch die Gesamtheit der Merkmale des Patentanspruches 1 gelöst. Weiterbildungen sind in den Unteransprüchen angegeben.

Bei der Erfindung ist das Mikroelektrodensystem als Dünneschicht-Vierpolsystem aufgebaut. Dabei umfasst der DNA-Chip ein Paar Polarisationselektroden zur Erzeugung eines elektromagnetischen Wechselfeldes und ein Paar Sensorelektroden zur Messung eines bindungsinduzierten Spannungsabfalls im Analyten. Die Polarisationselektroden sind dabei mit Wechselstrom bzw. -spannung gegebener Amplitude und gegebener Frequenz beaufschlagt. Mit Hilfe der Sensorelektroden und eines an sie angeschlossenen hochohmigen Messverstärkers lässt sich eine durch Bindungsereignisse hervorgerufene Widerstandänderung als Spannungsänderung polarisierungsfrei abgreifen. Der störende Einfluss der Elektrodenimpedanz ist somit eliminiert. Der Spannungsabgriff an den Sensorelektroden erfolgt hochohmig, so dass keine nennenswerten Ströme aus den Sensorelektroden aus- oder in diese eintreten. Auch findet hierdurch an den Sensorelektroden keine zusätzliche Polarisation statt, was die oben beschriebenen nachteiligen Effekte wie Polarisation, Filmbildung, Oxidation etc. minimiert. Diese Effekte können zwar nach wie vor an den Polarisationselektroden auftreten, gehen jedoch wegen der Spannungsmessung rein über die Sensorelektroden nicht oder nur wesentlich geringer in die Messergebnisse ein.

Durch die Trennung von polarisierenden Polarisationselektroden und messenden Sensorelektroden, an denen praktisch keine störenden chemischen oder physikalischen Vorgänge stattfinden, wird vorteilhafterweise die Qualität der Messung erheblich gesteigert. Dabei ist der Aufbau des Vierpolsystems mit Dünnschichtelektroden wesentlich, wobei solche Dünnschichtelektroden vorteilhafterweise durch die in der Halbleitertechnologie bekannten Verfahren in den Chip integriert werden können.

Bei einer bevorzugten Ausgestaltung der Erfindung weist der Träger des DNA-Chips ein Siliziumsubstrat auf, auf dem das Mikroelektrodensystem vorzugsweise in Dünnfilmtechnik integriert ist. Dabei sind die Elektroden direkt an eine sich im Si-Substrat befindliche integrierte Schaltung angeschlossen. Vorteilhaft dabei ist insbesondere das Fehlen von Zuleitungskapazitäten, welche sich vor allem bei Messungen im höheren Frequenzbereich, z.B. ab 10 MHz störend bemerkbar machen würden. Ein DNA-Chip der genannten Ausgestaltung ist somit auch bei hohen Messfrequenzen anwendbar.

Trotz der beschriebenen Maßnahmen sind noch weitere Parasitärkapazitäten zwischen den an der Messung beteiligten Elektroden vorhanden, die in ungünstigen Fällen störend bei der Impedanzmessung im Analyten sein können. Um hier Abhilfe zu schaffen, ist bei der Erfindung einer Sensorelektrode eine Schirmelektrode zugeordnet, die auf dem gleichen elektrischen Potential wie die Sensorelektrode gehalten ist. Vorzugsweise wird das elektrische Potential der Sensorelektrode an der Schirmelektrode durch einen an der Sensorelektrode angeschlossenen Pufferverstärker mit Verstärkung 1 gehalten. Der Pufferverstärker als aktives elektronisches Element entkoppelt Potential und Stromstärke, bzw. Ladung. Die zwischen den zusätzlichen Schirmelektroden und den anderen Elektroden auftretenden Parasitärkapazitäten sind so für die Messung elektrisch nicht wirksam, da das Laden oder Entladen der Kapazitäten vom aktiven Verstärker übernommen wird und so das Messgerät und die Sensorelektroden zur Potentialmessung entlastet sind. Schirmelektroden können z.B. beidseits der Sensorelektroden ausgeführt sein oder nur zwischen Sensorelektroden und Polarisationselektroden liegen. Die Effekte der Abschirmung durch die Schirmelektroden und die anwachsende Distanz einer Elektrodeneinheit durch Einfügen von Schirmelektroden sind im Einzelfall gegeneinander abzuwägen.

Der Pufferverstärker ist auf dem DNA-Chip integriert. Hierdurch ergibt sich ein besonders kompakter und wirkungsvoller Aufbau des DNA-Chips. Die Zuleitungen werden so kurz wie möglich gehalten und damit Störungen in sämtlichen Signalleitungen so gut wie möglich ausgeschlossen.

Um einen die Messung verfälschenden Stromfluss innerhalb der Sensorelektroden und auch der Schirmelektroden zu vermeiden, sollten diese Elektroden deutlich kleiner sein als die Potentialelektroden. Bei einer Breite der Potentialelektroden von 1 µm würde dies für die genannten Elektroden eine technisch nicht mehr realisierbare weitere Verkleinerung bedeuten. Bei einer bevorzugten Ausführungsform wird daher ein anderer Weg beschritten. Hier sind Sensor- und/oder Schirmelektroden gänzlich galvanisch vom Analyten getrennt und damit ein Stromfluss zwischen Analyt und Elektroden verhindert.

Bei einem anderen bevorzugten Ausführungsbeispiel wird dies durch eine punktförmige Ausgestaltung der Sensorelektroden erreicht. Dabei ist es erforderlich, dass eine Einrichtung zum Abgriff des Spannungsabfalls an den Sensorelektroden einen hohen Eingangswiderstand und eine niedrige Eingangskapazität aufweist. Die elektrische Verbindung der punktförmigen Sensorelektroden wird vorzugsweise durch eine im Substrat eingebettete ("vergrabene") Sammelleitung bewerkstelligt, die über Durchkontaktierungen mit den Sensorelektroden elektrisch verbunden ist.

Um die Tauglichkeit eines DNA-Chips als biochemisches Analysesystem zu gewährleisten, müssen die Elektrodengeometrien und damit der impedanzspektroskopische Erfassungsbereich möglichst an die Dimensionen biochemischer Moleküle angenähert werden. Eine Elektrodenbreiten von etwa 500 nm unterschreitende Miniaturisierung ist jedoch nur mit aufwendigster Technik zu sehr hohen Kosten realisierbar. Dabei kommt bei einem 4-Elektrodensystem erschwerend hinzu, dass der Abstand der Polarisationselektroden zueinander von Haus aus größer ist als bei einem 2-Elektrodensystem, weil ja zwischen den Polarisationselektroden weitere, z.B. die Sensorelektroden liegen. Bei einer besonders bevorzugten Ausführungsvariante ist nun eine das Mikroelektrodensystem einbettende Reaktionsschicht vorgesehen, welche den Raum, in dem Bindungsereignisse stattfinden und impedanzspektroskopisch erfasst werden können, wesentlich vergrößert. Mit der Reaktionsschicht lässt sich somit die Anzahl von erfassbaren Bindungsereignissen und damit der Messeffekt bzw. die Sensitivität eines DNA-Chips erheblich steigern.

Die Reaktionsschicht hat vorzugsweise eine Dicke, die mit der Breite der Elektroden korreliert ist, und entspricht vorteilhafterweise etwa dem 5-10fachen der Elektrodenbreite. In jedem Fall sollte die Reaktionsschicht eine Dicke unter 100 µm, da sich sonst zu lange Diffusionswege und damit verbunden zu lange Reaktionszeiten für den Transport der Zielmoleküle zu den Fängermolekülen ergeben würden. Geht man von 1 µm (1000 nm) als Elektrodenbreite aus, liegt die Dicke der Reaktionsschicht etwa 5 bis 10 µm. Damit ist gewährleistet, dass die in einer auf die Reaktionsschicht aufgebrachten Analytlösung enthaltenen Zielmoleküle mit ausreichender Geschwindigkeit eindiffundieren können. Als besonders geeignet haben sich Hydrogele zum Aufbau einer Reaktionsschicht herausgestellt.

Weitere Einzelheiten und Vorteile der Erfindung ergeben sich aus der nachfolgenden Figurenbeschreibung von Ausführungsbeispielen anhand der Zeichnung in Verbindung mit den Patentansprüchen. Es zeigen
- Fig. 1: die Draufsicht auf einen DNA-Chip mit Elektrodenmikrosystem in Interdigitalstruktur,
- Fig. 2: die Draufsicht auf ein Elektrodenmikrosystem mit vergrabenen Sammelleitungen,
- Fig. 3: die Draufsicht auf ein Elektrodenmikrosystem mit punktförmigen Sensorelektroden,
- Fig. 4: den Schnitt durch das Elektrodenmikrosystem aus Fig. 1 entlang der Linie IV-IV ohne (a) und mit (b) einer Reaktionsschicht,
- Fig. 5: den Schnitt durch das Elektrodenmikrosystem aus Fig. 1 entlang der Linie IV-IV mit auf Stegen liegenden und teilweise (a) bzw. ganz (b) vergrabenen Elektroden,
- Fig. 6: Elektrodenanordnungen in Draufsicht in Interdigitalstruktur (a) bzw. mit vergrabenen Elektrodensammelleitungen und Durchkontaktierungen (b),
- Fig. 7: Elektrodenanordnungen im Querschnitt mit Schirmelektroden und verschieden isolierten bzw. vergrabenen Elektroden (a) bis (e),
- Fig. 8: Elektrodenanordnungen im Querschnitt mit übereinander liegenden Polarisations- und Sensorelektroden in verschiedenen Ausführungsformen (a) bis (f),
- Fig. 9: Elektrodenanordnungen im Querschnitt mit Beispielen von Kombinationen der unterschiedlichen Anordnungstechniken (a) bis (d), jeweils in Prinzipdarstellung.

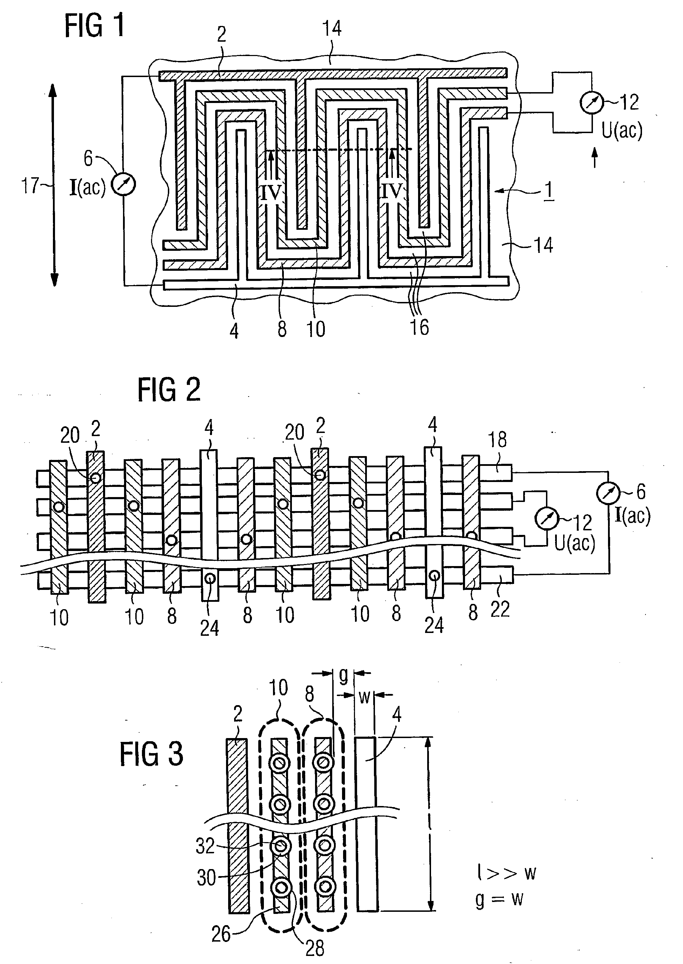

Fig. 1 zeigt einen Ausschnitt eines DNA-Chips in Draufsicht, wobei ein Träger 14 angedeutet ist: Der Ausschnitt zeigt einen Spot 1. Dessen Mikroelektrodensystem zeigt ein Paar Polarisationselektroden 2 und 4. Die Polarisationselektroden 2 und 4 sind von einer Stromquelle 6 gespeist. Die Stromquelle 6 erzeugt einen durch die Elektroden 2 und 4 fließenden Wechselstrom, der einen sich über der Elektrodenanordnung befindenden, nicht im Einzelnen dargestellten Analyten polarisiert. Der Analyt schließt also den Stromkreis über Stromquelle 6 und Polarisationselektroden 2 und 4.

Weiterhin ist in Fig. 1 ein Paar Sensorelektroden 8 und 10 dargestellt, die an ein hochohmiges Voltmeter 12 angeschlossen sind. Da sich zwischen den beiden Polarisationselektroden 2 und 4 in einem auf den Träger 14 und die Elektroden aufgebrachten elektrisch leitfähigen Analyten Stromlinien bilden, liegen die Sensorelektroden 8 und 10 mit Bereichen des Analyten unterschiedlichen elektrischen Potentials in Berührung. Diese Potentialdifferenz ist dann am Voltmeter 12 angezeigt. Da das Voltmeter 12 einen hochohmigen Eingangswiderstand besitzt, findet jedoch zwischen Analyt und den Sensorelektroden 8 und 10 kein nennenswerter Ladungstransport, also kein nennenswerter Stromfluss statt.

In Figur 1 sind alle vier Elektroden 2, 4, 8 und 10 in Fig. 1 in Interdigitalstruktur angeordnet. Die dargestellte Elektrodenanordnung ist als Teil eines Mikrochips zum Beispiel durch Benutzung der Dünnfilmtechnik auf einem Träger 14, zum Beispiel Silizium mit einer elektrisch isolierenden Deckschicht (nicht dargestellt), z.B. Siliziumoxid, aufgebracht. Alle Elektroden sind durch elektrisch isolierende Zwischenräume 16 voneinander getrennt. Die symbolisch dargestellten Stromquel-le 6 und das Voltmeter 12 sind i.A. als integrierte elektrische Schaltungen im Träger 14 z.B. unterhalb der Elektroden ausgeführt. Die Anordnung ist in Ausdehnungsrichtung 17 stark gestaucht dargestellt. Gleiches gilt für die Figuren 2,3 und 6.

Die in Figur 2 dargestellte Ausführungsform beinhaltet ebenfalls ein Paar Polarisationselektroden 2 und 4 und ein Paar Sensorelektroden 8 und 10. Die einzelnen Abschnitte der Elektroden sind jedoch nicht wie in Fig. 1 kamm- bzw. mäanderförmig ineinander gefaltet. Jeder Elektrodentyp ist jeweils als eine Mehrzahl voneinander an sich elektrisch isolierter länglicher Streifen auf der Chipoberfläche ausgeführt. So sind je zwei Streifen der Polarisationselektroden 2 und 4 und je 4 Streifen der Sensorelektroden 8 und 10 zu sehen.

Zur elektrischen Verbindung ist hier jedem Elektrodentyp eine Elektrodensammelleitung zugeordnet. Der Polarisationselektrode 2 ist zum Beispiel die Sammelleitung 18 zugeordnet. Die beiden Streifen der Polarisationselektrode 2 sind deshalb mit Hilfe von elektrischen Durchkontaktierungen 20 mit der ihr zugeordneten Sammelleitung 18 verbunden. Gegenüber allen anderen Elektroden 4, 8 und 10 ist die Sammelleitung 18 elektrisch isoliert. Deshalb liegen Sammelleitungen zum Beispiel vergraben in der Isolierenden Deckschicht 36 des Trägers 14 oder sind zumindest sowohl gegeneinander als auch gegenüber allen Elektroden anderweitig elektrisch isoliert. Der zweiten Polarisationselektrode 4 ist die Sammelleitung 22 mit entsprechenden Durchkontaktierungen 24 zugeordnet usw.

In Fig. 3 ist wie aus den Figuren 1 und 2 zu erkennen, dass die geometrischen Abmessungen Breite w und Abstand g der Polarisations- und Sensorelektroden in etwa gleich groß sind. Dies kann eventuell zu Problemen führen, da bei einem vierpoligen Messverfahren üblicherweise die Sensorelektroden stets wesentlich kleiner als die Polarisationselektroden ausgeführt sind. In Figur 3 ist deshalb eine alternative Ausführungsform von Sensorelektroden dargestellt. Die Sensorelektrode 10 enthält eine Sammelleitung 26, die entsprechend den Sammelleitungen 18 und 22 in Figur 2 im Substrat vergraben bzw. genügend elektrisch isoliert von z.B. anderen Leitungen und dem Analyten ausgeführt ist. Der Kontakt zwischen der Sensorelektrode 10 und dem Analyten wird nicht mehr flächig wie in Fig. 1 und 2, sondern nur noch über punktförmige Einzelelektroden 28 hergestellt.

Jede Einzelelektrode 28 enthält einen punktförmigen Elektrodenkopf 30, der mit Hilfe einer elektrischen Durchkontaktierung 32 mit der Elektrodensammelleitung 26 verbunden ist. Obwohl die Elektrodenbreite w und der Elektrodenabstand g gleich sind, wird durch diese Maßnahme erreicht, dass die effektive Elektrodenfläche der Sensorelektroden 8 und 10 gegenüber der Fläche der Polarisationselektroden 2 und 4 wesentlich kleiner ist. Hier ist nochmals angedeutet, dass in realen Anordnungen die "Länge" 1 der Elektrode wesentlich größer als deren Breite w ist.

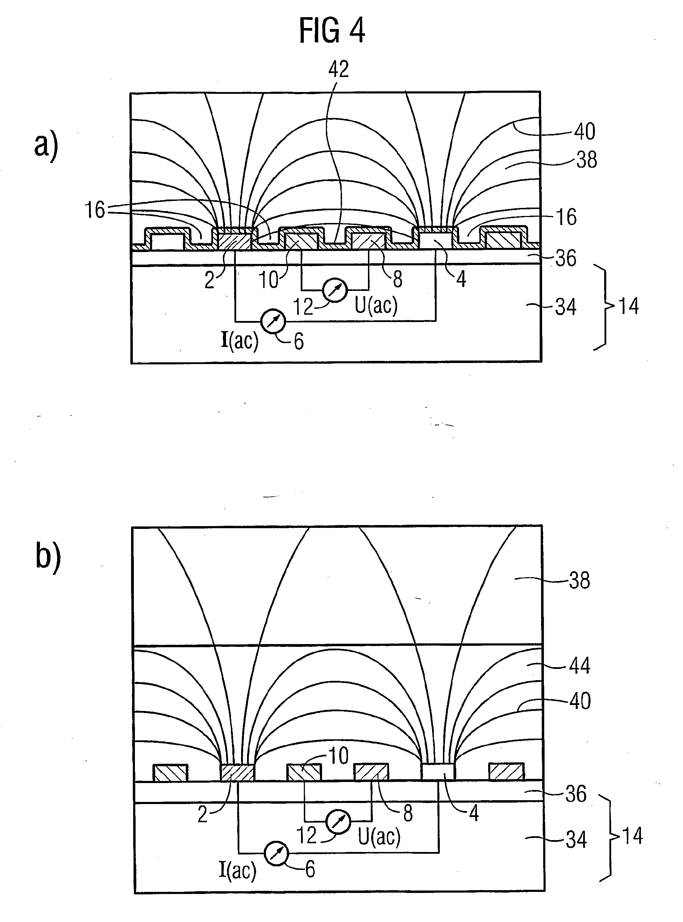

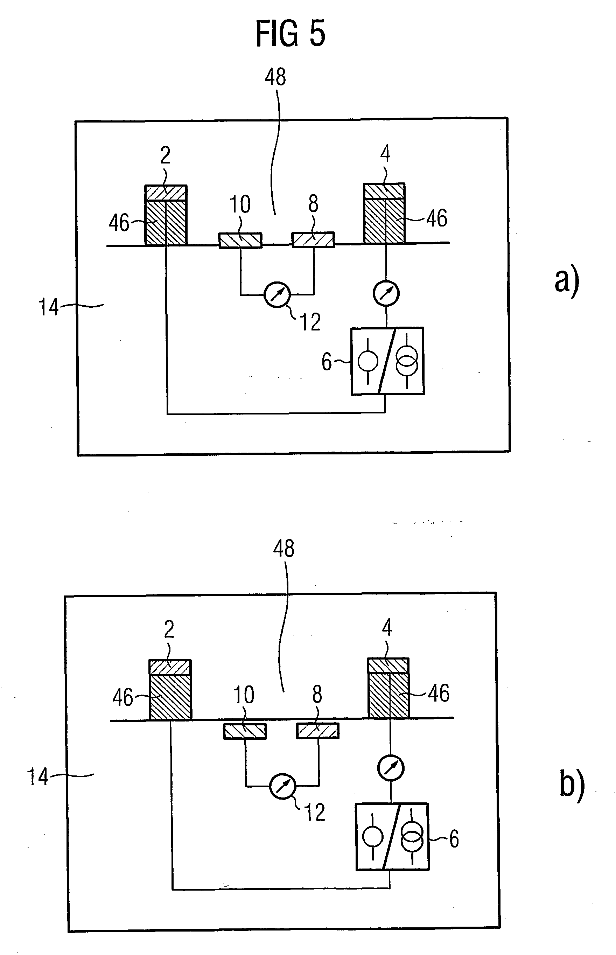

Ein Schnitt durch die Figur 1 entlang der Linie IV-IV ist in den Figuren 4 und 5 dargestellt. In Fig. 4a ist der Aufbau des Trägers 14 aus dem eigentlichen in der Regel einkristallinen Chipträger, also dem Substrat 34, z.B. Silizium, und einer elektrisch isolierenden Schicht 36, z.B. Siliziumoxid, dargestellt. Durch die isolierende Schicht 36 und die Zwischenräume 16 sind die Elektroden 2, 4, 8 und 10 voneinander elektrisch isoliert. Der Analyt 38, der mit der Chipoberfläche und den Elektroden in Kontakt steht, ist von Stromlinien 40 durchsetzt, die in den Polarisationselektroden 2 und 4 enden. Diese Stromlinien 40 entstehen durch die Einspeisung des Polarisationsstromes von der Stromquelle 6 vermittels der Polarisationselektroden 2 und 4 in den Analyten 38.

Bei letzterer Anordnung können Fängermoleküle in einer sehr dünnen Schicht 42 direkt auf der Chipoberfläche angebracht werden. Methoden hierzu sind aus dem Stand der Technik hinreichend bekannt. Ein Zielmolekül aus dem Analyten 38 kann zum Fängermolekül in der Schicht 42 diffundieren. Nur in der Schicht 42 können so Bindungsereignisse überhaupt stattfinden und nur dort detektiert werden. Die Detektion findet statt, weil in der Schicht 42 sich die elektrischen Eigenschaften des Mediums aufgrund der Bindungsereignisse ändern. Die Strom- und Spannungsverhältnisse im Analyten 38 ändern sich und an den Sensorelektroden 8 und 10 wird eine entsprechend geänderte Spannung abgegriffen. Wie aus dem Verlauf der Stromlinien 40 ersichtlich, wird jedoch nur ein sehr geringer Anteil des felddurchsetzten Raumes zur Reaktion benutzt. Die in einer derartigen Anordnung auftretenden Veränderungen der elektrischen Eigenschaften des Analyten und somit die detektierbaren Spannungen zwischen den Sensorelektroden 8 und 10 können gegebenenfalls unterhalb des messtechnisch erfassbaren Bereiches von Messspannungen liegen.

Eine Verbesserung kann durch das Aufbringen einer Reaktionsschicht 44 auf den DNA-Chip stattfinden, wie in Fig. 4b gezeigt. In dieser Reaktionsschicht 44, zum Beispiel einem Hydrogel, können in deren gesamten Volumen Fängermoleküle eingebettet bzw. fixiert werden. Die Reaktionsschicht 44 ist nur etwa 5-10 µm dick ausgeführt. Somit kann eine chemische Reaktion zwischen Zielmolekülen aus dem Analyten 38, die in die sehr dünne Reaktionsschicht 44 schnell eindiffundieren können, und den Fängermolekülen stattfinden. Die chemische Reaktion bzw. Veränderung elektrischer Parameter in der Reaktionsschicht 44 wird somit von einem wesentlich größeren Bereich von Stromlinien 40 durchdrungen. Bei einer Reaktion werden deshalb wesentlich größere Spannungen am Voltmeter 12 gemessen.

In den Figuren 5a und 5b liegen die Polarisationselektroden 2 und 4 auf elektrisch isolierenden Stegen 46 beabstandet zur Oberfläche des Trägers 14. Dies kann in bestimmten Fällen, das heißt für bestimmte Kombinationen aus immobilisierter Spezies und Analyt, zu einer günstigeren Feldverteilung im Analytraum 48 führen. Günstiger bedeutet, dass zwischen den Sensorelektroden 8 und 10 eine möglichst große Spannungsdifferenz am Voltmeter 12 gemessen werden kann.

In Figur 5a sind die beiden Sensorelektroden 8 und 10 teilweise im Träger 14 vergraben. In Figur 5b sind diese beiden Elektroden vollständig vergraben und somit galvanisch vom Analytraum 48 getrennt. Der Grad der Eingrabung oder die galvanische Trennung können je nach Analyt 38 und immobilisierter Spezies günstiger oder ungünstiger für das Erzielen zuverlässiger Messergebnisse am Voltmeter 12 sein. Auch die Auswahl von Stegen 46 als Träger für die Polarisationselektroden 2 und 4 kann je nach Fall günstiger oder ungünstiger sein. Eine entsprechende Auswahl der strukturellen Anordnung ist in den meisten Fällen experimentell zu finden.

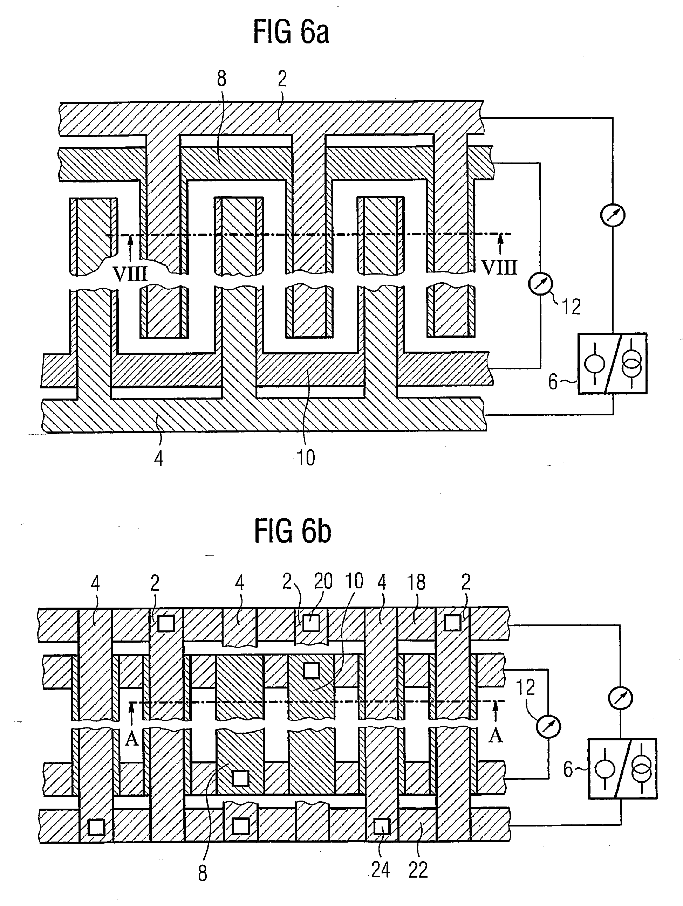

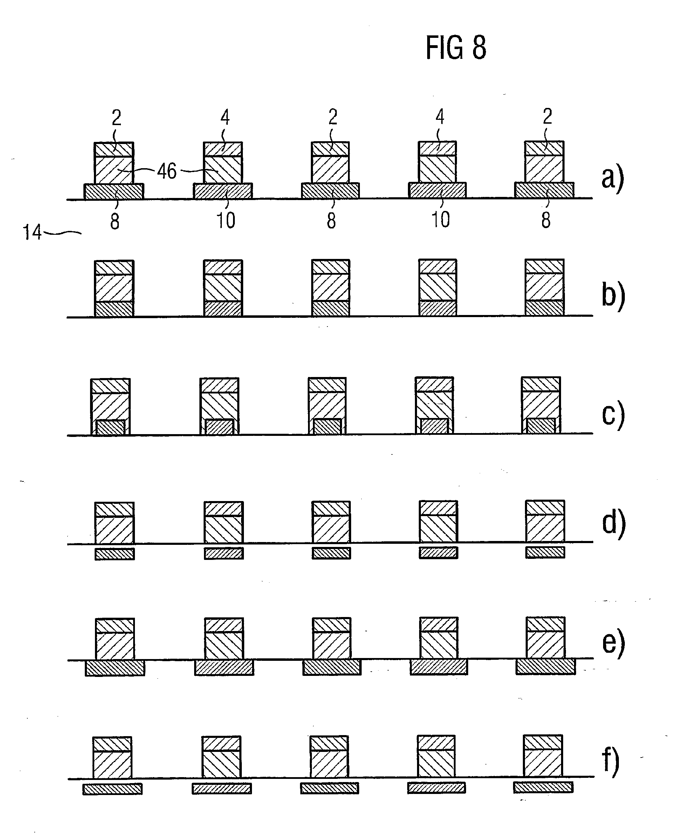

Die Figuren 6a und 6b zeigen nochmals verschiedene Ausführungsformen für die Gestaltung bzw. für die Verbindungstechnik der verschiedenen Elektroden bezüglich des Layouts und der verwendeten Technologien im Halbleiterprozess bei der Chipherstellung. Fig. 6a entspricht bezüglich der Polarisationselektroden 2 und 4 der Ausführungsform in Fig. 1. Allerdings liegen hier die Sensorelektroden 8 und 10 in etwa gleichem Layout wie die Polarisationselektroden 2 und 4, aber unter diesen, getrennt durch eine elektrisch isolierende Zwischenschicht, also Stege 46. Ein Schnitt durch diese Anordnung ist in Fig. 8 dargestellt. Durch einen derartigen Aufbau des DNA-Chips wird ein sehr kompakter Aufbau erreicht, der für beide Elektrodentypen, also Sensor- und Polarisationselektroden 4 und 2 nahezu die kleinstmögliche, im Halbleiterprozess erreichbare Strukturgröße und Abstände zueinander erlaubt. Außerdem werden durch den Aufbau von übereinander angeordneten Elektroden das effektive Verhältnis zwischen der Größe beider Elektrodentypen ähnlich wie bei der in Fig. 3 gezeigten Ausgestaltung beeinflusst, die Sensorelektroden 8 und 10 zeigen also eine geringe Größe im Verhältnis zu den Polarisationselektroden 2 und 4.

Fig. 6b entspricht der in Fig. 2 dargestellten Anordnung, nur dass auch hier, wie in Fig. 6a, die Elektrodentypen übereinander, anstatt nebeneinander angeordnet sind. Auch bei der in Fig. 6b dargestellten Ausgestaltung liegen die Polarisationselektroden 2 und 4 wieder auf den elektrisch isolierenden Stegen 46 über den Sensorelektroden 10 und 8, wobei die Stege aus der Draufsicht nicht ersichtlich sind. Ein Schnitt entlang der Linie A-A durch die Anordnung wie in Fig. 6a entspricht auch hier der Figur 8, nur dass die übereinanderliegenden Elektroden vertauscht sind, also die Polarisationselektroden 2 bzw. 4 über den Sensorelektroden 10 bzw. 8 liegen.

Die Auswahl der verwendeten Technologien bzw. Layouts richtet sich meist nach den Gegebenheiten bzw. Randbedingungen des zu verwendenden Halbleiterprozesses und der mit dem DNA-Chip zu lösenden Aufgabe. Natürlich können auch Kombinationsmöglichkeiten aus den vorgestellten Techniken verwendet werden. So ist es zum Beispiel denkbar, die Polarisationselektroden gemäß Fig. 6a in kammförmiger Interdigitalstruktur auszuführen und die Sensorelektroden gemäß Figur 6b als zu Sammelleitungen durchkontaktierte Einzelstreifen auszuführen und beide Anordnungen durch Stege 46 zu trennen und übereinander anzuordnen.

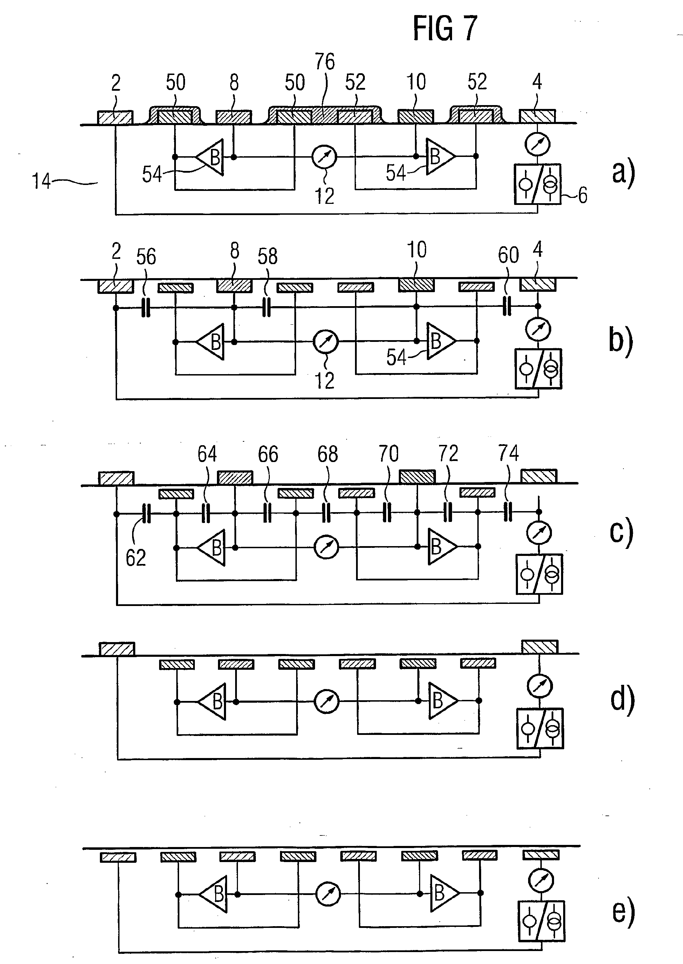

In der Figur 7 sind jeder Sensorelektrode 8 und 10 je zwei Schirmelektroden 50 und 52 zugeordnet. Durch Pufferverstärker 54 ist das jeweilige elektrische Potential der Sensorelektroden 8 und 10 an den Schirmelektroden 50 und 52 elektrisch aktiv gehalten. Die im Fall fehlender Schirmelektroden wirksamen Parasitärkapazitäten 56, 58 und 60 zwischen Sensorelektroden 8 und 10 und Polarisationselektroden 2 und 4 werden durch diese Maßnahme teils elektrisch unwirksam, wie sich leicht aus einem nicht dargestellten elektrischen Ersatzschaltbild der Anordnung ergibt. Mit vorhandenen Schirmelektroden bilden sich nämlich die parasitären Kapazitäten 62 bis 74 aus. Hiervon sind aber nur die Kapazitäten 62, 68 und 74 für die Messung in den Messgeräten Voltmeter 12 und dem der Stromquelle 6 zugeordneten Strommesser wirksam. Die anderen Kapazitäten sind über die Pufferverstärker 54 versorgt, werden also über diese ge- bzw. entladen und gehen somit nicht in die Messung mit ein, was i. A. zu einem wesentlich verbesserten Messergebnis führt. Somit können sich diese parasitären Kapazitäten nicht mehr nachteilig auf die Impedanz zwischen den Sensor- und Polarisationselektroden auswirken.

Die Pufferverstärker 54 können mit_Hilfe der Halbleitertechnologie direkt im Träger 14 unterhalb der Elektrodenanordnung realisiert werden. Hierdurch werden Signalwege verkürzt, zusätzliche Kapazitäten so klein wie möglich gehalten, und somit die Frequenzeigenschaften der gesamten Messanordnung günstig beeinflusst. Die verschiedenen in Figur 7 dargestellten Ausführungsformen von Anordnung der einzelnen Elektroden unterscheiden sich dadurch, dass eine oder mehrere Elektroden galvanisch vom Analyten getrennt sind bzw. ganz oder teilweise im Substrat vergraben sind. Die galvanische Trennung erfolgt, wie in Fig. 7a z.B. durch Aufoxidieren einer zusätzlichen Oxidschicht über den Elektroden oder durch Vergraben der Elektroden, wie in Fig. 7e für alle beteiligten Elektroden durchgeführt. Auch in Fig. 7 sind wieder verschiedene Kombinationen zwischen den dargestellten Alternativen denkbar. Diese können durch Simulationen oder im Experiment an die Bedürfnisse einer speziellen Messung bzw. Ausgestaltung des Chips angepasst werden.

Auch in Figur 8 sind verschiedenste Kombinationen der Anordnung aus Sensor- und Polarisationselektroden dargestellt. Während die Polarisationselektroden stets auf Stegen über dem Träger ruhen, sind die Sensorelektroden jeweils unterhalb oder innerhalb der Stege angeordnet. Durch diese Maßnahmen kann eine äußerst kompakte Bauweise des DNA- Chips erreicht werden, da die Abstände zwischen den Elektrodentypen minimiert werden können.

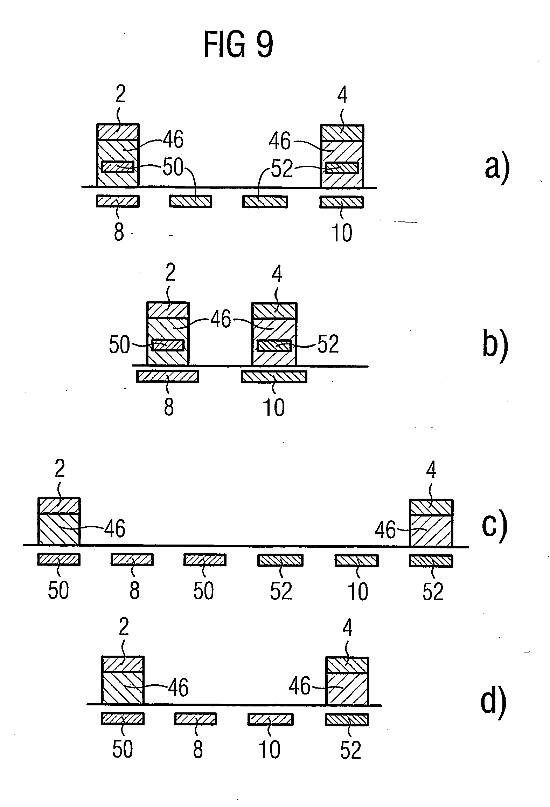

Figur 9 zeigt Konfigurationen aus Elektroden entsprechend den Figuren 8 wobei hier zusätzlich eine oder mehrere Schirmelektroden zwischen Sensorelektroden untereinander oder zwischen Sensor- und Polarisationselektroden vorgesehen sind. Auch für die in Figur 9 dargestellten Anordnungen gilt bezüglich der Auswahl einer bestimmten Konfiguration das oben Gesagte.

Die in den Figuren dargestellten Ausführungsformen sind Beispiele für die verschiedenen Kombinationsmöglichkeiten aus den aufgeführten Techniken. Natürlich sind, wie mehrfach erwähnt auch andere als die explizit dargestellten Kombinationen möglich.

## Patentansprüche

1. DNA-Chip mit einem Träger (14) und einem darauf angeordneten Mikroarray von immobilisierte Fängermoleküle enthaltenden Spots (1), wobei jeder Spot (1) ein Mikroelektrodensystem zur impedanzspektroskopischen Detektion von Bindungsereignissen zwischen den Fängermolekülen und Zielmolekülen einer auf die Spots (1) applizierten Analytlösung (38) enthält, wobei das Mikroelektrodensystem ein Dünnschicht-Vierpolsystem ist und wobei das Dünnschicht-Vierpolsystem zwei Polarisationselektroden (2, 4) zur Erzeugung eines elektromagnetischen Wechselfeldes und zwei Sensorelektroden (8, 10) zur Messung eines Spannungsabfalls im Analyten (38) umfasst und wenigstens einer Sensorelektrode (8, 10) eine Schirmelektrode (50, 52) zugeordnet ist, die auf dem gleichen elektrischen Potential wie die Sensorelektrode (8, 10) gehalten ist.

2. DNA-Chip nach Anspruch 1, **dadurch gekennzeichnet, dass** der Träger (14) ein Siliziumsubstrat (34) umfasst, auf dem das Mikroelektrodensystem in Dünnfilmtechnik integriert ist.

3. DNA-Chip nach einem der Ansprüche 1 oder 2, **dadurch gekennzeichnet, dass** von einem an die Sensorelektrode (8, 10) angeschlossenen Pufferverstärker (54) mit verstärkung (1) das elektrische Potential der Sensorelektrode (8, 10) an der Schirmelektrode (50, 52) gehalten ist.

4. DNA-Chip nach Anspruch 3, **dadurch gekennzeichnet, dass** der Pufferverstärker (54) auf dem Träger (14) integriert ist.

5. DNA-Chip nach einem der vorhergehenden Ansprüche, **dadurch gekennzeichnet, dass** wenigstens eine sensorelektrode (8, 10) und/oder wenigstens eine Schirmelektrode (50, 52) galvanisch vom Analyten (38) getrennt sind.

6. DNA-Chip nach einem der vorhergehenden Ansprüche, **dadurch gekennzeichnet, dass** eine Sensorelektrode (8, 10) punktförmige Einzelelektroden (28) enthält, die mittels Durchkontaktierungen (32) mit einer vergrabenen Elektrodensammelleitung (26) elektrisch verbunden sind.

7. DNA-Chip nach einem der vorhergehenden Ansprüche, **dadurch gekennzeichnet, dass** das Dünnschicht-Mikroelektrodensystem in einer Fängermoleküle enthaltenden Reaktionsschicht (44) eingebettet ist.

8. DNA-Chip nach Anspruch 7, **dadurch gekennzeichnet, dass** die Dicke der Reaktionsschicht (44) unter 100 µm beträgt und mit der Breite der Elektroden bzw. deren Zwischenräume korreliert ist.

9. DNA-Chip nach Anspruch 8, wobei die Breite der Elektroden etwa 1 µm beträgt, **dadurch gekennzeichnet, dass** die Dicke der Reaktionsschicht (44) etwa dem 5-10fachen Wert der Elektrodenbreite entspricht.

10. DNA-Chip nach Anspruch 7 oder 8, **dadurch gekennzeichnet, dass** die Reaktionsschicht (44) ein Hydrogel ist.

11. DNA-Chip nach einem der vorhergehenden Ansprüche, **dadurch gekennzeichnet, dass** das Dünnschicht-Vierpolsystem eine Interdigital-Stromelektrodenanordnung mit Doppelmäanderstromabgriffen bildet.

## Claims

1. DNA chip comprising a carrier (14) and, arranged thereon, a microarray of spots (1) containing immobilized catcher molecules, each spot (1) containing a microelectrode system for the impedance-spectroscopic detection of binding events between the catcher molecules and target molecules of an analyte solution (38) applied to the spots (1), the microelectrode system being a thin-film four-pole system, and the thin-film four-pole system comprising two polarization electrodes (2, 4) for generating an alternating electromagnetic field and two sensor electrodes (8, 10) for measuring a voltage drop in the analyte (38), and at least one sensor electrode (8, 10) being assigned a shielding electrode (50, 52), which is held at the same electrical potential as the sensor electrode (8, 10).

2. DNA chip according to Claim 1, **characterized in that** the carrier (14) comprises a silicon substrate (34), on which the microelectrode system is integrated using thin-film technology.

3. DNA chip according to either of Claims 1 and 2, **characterized in that** the electrical potential of the sensor electrode (8, 10) is held at the shielding electrode (50, 52) by a buffer amplifier (54) connected to the sensor electrode (8, 10) and having a gain of 1.

4. DNA chip according to Claim 3, **characterized in that** the buffer amplifier (54) is integrated on the carrier (14).

5. DNA chip according to one of the preceding claims, **characterized in that** at least one sensor electrode (8, 10) and/or at least one shielding electrode (50, 52) are directly electrically isolated from the analyte (38).

6. DNA chip according to one of the preceding claims, **characterized in that** a sensor electrode (8, 10) contains pointlike individual electrodes (28) which are electrically connected to a buried electrode collective line (26) by means of plated-through holes (32).

7. DNA chip according to one of the preceding claims, **characterized in that** the thin-film microelectrode system is embedded in a reaction layer (44) containing catcher molecules.

8. DNA chip according to Claim 7, **characterized in that** the thickness of the reaction layer (44) is less than 100 µm and is correlated with the width of the electrodes or the interspaces thereof.

9. DNA chip according to Claim 8, the width of the electrodes being approximately 1 µm, **characterized in that** the thickness of the reaction layer (44) corresponds to approximately 5-10 times the value of the electrode width.

10. DNA chip according to Claim 7 or 8, **characterized in that** the reaction layer (44) is a hydrogel.

11. DNA chip according to one of the preceding claims, **characterized in that** the thin-film four-pole system forms an interdigital current electrode arrangement with double meandering current taps.

## Revendications

1. Puce à ADN ayant un support ( 4 ) et un microréseau, qui y est mis dessus, de tâches ( 1 ) contenant des molécules de capture immobilisées, chaque tâche ( 1 ) contenant un système de microélectrodes pour la détection spectroscopique par impédance d'évènements de liaison entre les molécules de capture et les molécules cibles d'une solution ( 38 ) d'analyte appliquée sur la tâche, dans laquelle le système de microélectrodes est un système quadripolaire à couche mince et dans lequel le système quadripolaire à couche mince comprend deux électrodes ( 2,4 ) de polarisation pour la production d'un champs alternatif électromagnétique et deux électrodes ( 8,10 ) de capteur pour la mesure d'une chute de tension dans l'analyte ( 38 ) et à au moins l'une des électrodes ( 8,10 ) de capteur est associée une électrode ( 50,52 ) de protection, qui est maintenue au même potentiel électrique que l'électrode ( 8,10 ) de capteur.

2. Puce à ADN suivant la revendication 1,
**caractérisée en ce que** le support ( 14 ) comprend un substrat ( 34 ) en silicium, sur lequel le système de microélectrodes est intégré en technique à couche mince.

3. Puce à ADN suivant l'une des revendications 1 ou 2,
**caractérisée en ce que** le potentiel électrique de l'électrode ( 8,10 ) de capteur est maintenu sur l'électrode ( 50,52 ) de protection avec amplification ( 1 ) du potentiel électrique par un amplificateur ( 54 ) tampon raccordé à l'électrode ( 8,10 ) de capteur.

4. Puce à ADN suivant la revendication 3,
**caractérisée en ce que** l'amplificateur ( 54 ) tampon est intégré sur le support ( 14 ).

5. Puce à ADN suivant l'une des revendications précédentes,
**caractérisée en ce qu'**au moins une électrode ( 8,10 ) de capteur et/ou au moins une électrode ( 50,52 ) de protection sont séparées galvaniquement de l'analyte ( 38 ).

6. Puce à ADN suivant l'une des revendications précédentes,
**caractérisée en ce qu'**une électrode ( 8,10 ) de capteur comporte des électrodes ( 28 ) individuelles ponctuelles, qui sont reliées électriquement au moyen de trous ( 32 ) métallisés à un conducteur ( 26 ) collecteur d'électrode enterré.

7. Puce à ADN suivant l'une des revendications précédentes,
**caractérisée en ce que** le système de microélectrodes en couche mince est incorporé dans une couche ( 44 ) de réaction contenant des molécules de capture.

8. Puce à ADN suivant la revendication 7,
**caractérisée en ce que** l'épaisseur de la couche ( 44 ) de réaction est inférieure à 100 µm et est en corrélation avec la largeur des électrodes ou de leurs intervalles.

9. Puce à ADN suivant la revendication 8,
dans laquelle la largeur des électrodes est d'environ 1 µm, **caractérisée en ce que** l'épaisseur de la couche ( 44 ) de réaction correspond à environ 5 à 10 fois la largeur des électrodes.

10. Puce à ADN suivant l'une des revendications 7 ou 8,
**caractérisée en ce que** la couche ( 44 ) de réaction est un hydrogel.

11. Puce à ADN suivant l'une des revendications précédentes,
**caractérisée en ce que** le système quadripolaire en couche mince forme un agencement d'électrode de courant interdigité ayant des prises de courant sinueuses doubles.
